# EUROPÄISCHE PATENTSCHRIFT

(11) **EP 2 642 926 B1**
(45) Veröffentlichungstag und Bekanntmachung des Hinweises auf die Patenterteilung: **10.05.2017**
(21) Anmeldenummer: 11801622.9
(22) Anmeldetag: 23.11.2011
(51) Int. Cl.: A61B 6/03, G21F 3/00, A61N 5/00, A61B 6/10, A61N 5/10

(54) **STRAHLENSCHUTZVORRICHTUNG**
RADIATION PROTECTION DEVICE
DISPOSITIF DE PROTECTION CONTRE LE RAYONNEMENT

(30) Priorität: 24.11.2010 DE 102010061893
(43) Veröffentlichungstag der Anmeldung: 02.10.2013
(73) Patentinhaber: MAVIG GmbH, 81829 München (DE)
(72) Erfinder: MURASE, Sosuke, 81929 München (DE); BALLSIEPER, Barbara, 81829 München (DE)
(74) Vertreter: Vossius & Partner Patentanwälte Rechtsanwälte mbB
(86) Internationale Anmeldenummer: PCT/EP2011/070790
(87) Internationale Veröffentlichungsnummer: WO 2012/069528

(56) Entgegenhaltungen:
- DE-A1-102004 039 411
- DE-U1- 20 307 606
- GB-A- 2 461 569

## Beschreibung

### Technisches Gebiet

Die Erfindung betrifft eine Strahlenschutzvorrichtung wie sie zur Abschirmung einer von einer Strahlungsquelle abgestrahlten Strahlung, insbesondere einer Röntgenstrahlung beispielsweise bei einer Computertomographie, eingesetzt werden kann.

### Stand der Technik

In der Medizin sowie auch in verschiedenen anderen Fachgebieten werden heutzutage verschiedenartige bildgebende Verfahren zur Untersuchung von inneren und äußeren Strukturen eines Objekts wie insbesondere von Organen und Strukturen des menschlichen Körpers eingesetzt. Unter anderem werden dabei tomografische Verfahren wie die Computertomografie, die Sonografie, die Magnetresonanztomografie, die Positronen-Emissions-Tomografie oder ähnliche Verfahren als schnittbildgebende Verfahren eingesetzt.

Dabei wird beispielsweise unter Computertomographie die computergestützte Auswertung von Röntgenaufnahmen eines Objekts, die aus verschiedenen Richtungen aufgenommen sind, verstanden, wobei ein dreidimensionales Bild des Objekts und insbesondere auch seines Inneren erzeugt wird. Typischerweise werden bei der Computertomographie Röntgenstrahlen gezielt abgegeben, so dass sie ein Objekt durchdringen und von Detektoren gleichzeitig aufgezeichnet werden. Die Auswertung der abgegebenen Strahlungsintensität und der detektierten Strahlungsintensität liefert Informationen bezüglich der Absorption der Röntgenstrahlung durch das Objekt. Der Grad der Absorption wird dabei meistens in Grauwerten oder eingefärbt dargestellt und auf einer Skala wie beispielsweise der Hounsfield-Skala angegeben. In einem Computer werden dann diesbezügliche Daten ausgewertet und zu Schnittbildern beziehungsweise Serien von Schnittbildern sowie zu 3D-Ansichten in beliebigen Ebenen verarbeitet. Verschiedene Gewebearten des Objekts können in den Schnittbildern und Ansichten erkannt werden, da jedes Gewebe je nach seiner Beschaffenheit eine bestimmte Graustufe beziehungsweise Färbung aufweist.

Tomografische Verfahren werden heute insbesondere in der Medizin auch während Interventionen am Objekt eingesetzt, wobei das Objekt und dessen Inneres während der Intervention laufend visualisiert werden. Dies ermöglicht dem Operateur beziehungsweise dem Arzt, die Intervention optisch zu überwachen und exakt auszuführen, ohne das Objekt entsprechend öffnen zu müssen. Interventionen in diesem Zusammenhang umfassen beispielsweise die Punktion von Gelenken oder Organen zu diagnostischen oder therapeutischen Zwecken, die Drainage von Innenräumen des Objekts beziehungsweise des menschlichen Körpers sowie Ähnliches.

Zur Durchführung tomografischer Verfahren werden heute verschiedenartige Scanner beziehungsweise Detektoren verwendet, die typischerweise einen Tunnel aufweisen, in den das zu untersuchende Objekt zumindest teilweise eingeführt wird. Im Tunnel wird das Objekt dann bestrahlt und analysiert. Zumindest bei den Tunnelöffnungen besteht bei diesen Detektoren häufig das Risiko, dass Strahlung von verhältnismäßig hoher Intensität nach außen durch die Tunnelöffnung austritt. Diese Strahlung kann direkt vom Scanner beziehungsweise Detektor abgestrahlte Primärstrahlung umfassen sowie auch durch das Objekt gestreute und von diesem abgestrahlte Sekundärstrahlung. Auf Grund der bekanntermaßen gesundheitsschädigenden Wirkung solcher Strahlung und insbesondere von Röntgenstrahlung, die abhängig ist von der Expositionsdauer und Strahlungsintensität, werden bei der Durchführung beispielsweise der Computertomographie üblicherweise Maßnahmen ergriffen, um den Operateur beziehungsweise den Arzt vor der Strahlung zu schützen. Dabei wird häufig Schutzkleidung eingesetzt, die einen mit einem Schwermetall wie Blei versehenen textilen Stoff oder Kunststoff umfasst. Da die Schutzwirkung mit zunehmendem Schwermetallgehalt der Schutzkleidung zunimmt, sind solche Schutzkleidungen typischerweise verhältnismäßig schwer, was die Bewegungsfreiheit des Operateurs beeinträchtigt und seine physische Belastung erhöht. Insbesondere bei den erwähnten Interventionen kann auch die Bewegungsfreiheit des Operateurs von Bedeutung sein. Zudem werden häufig schürzenförmige Schutzkleidungen verwendet, die nicht den ganzen Körper abdecken, so dass bestimmte Körperpartien wie die Schultern oder der Kopf und insbesondere die speziell strahlenempfindlichen Augen nicht geschützt sind.

Als Alternative oder als Ergänzung zu den erwähnten Schutzkleidungen sind heute verschiedene zusätzliche Strahlenschutzvorrichtungen bekannt. Beispielsweise zeigt die US 4,977,585 A einen Röntgenscanner mit einem Tunnel zum Einführen eines Patienten in den Scanner, der an beiden Öffnungen des Tunnels Abschirmvorhänge aufweist. Die Abschirmvorhänge können die Öffnungen bis zum Patienten hin flexibel abdecken und weisen eingebettetes Blei oder Messing auf. Somit kann die Strahlung vom Scanner aus nach außen hin abgeschirmt werden. Da jedoch ein Zugriff beispielsweise mittels der Hände eines Operateurs in den Innenraum des Tunnels ohne Öffnen oder Verschieben der Abschirmvorhänge nicht vorgesehen ist, sind Interventionen der oben beschriebenen Art bei diesem Röntgenscanner nur unter verhältnismäßig starker Beeinträchtigung der Schutzwirkung der Abschirmvorhänge möglich. Zudem ist auch die Sicht in den Innenraum des Röntgenscanners durch die Abschirmvorhänge eingeschränkt, was Interventionen durch den Operateur erschweren kann.

Weiter ist beispielsweise in der WO 2007/060561 A1 ein Strahlenschild für einen tomografischen Scanner gezeigt. Der Strahlenschild ist zylindersegmentförmig ausgestaltet und um den Scanner herum schwenkbar, so dass er zwischen dem Scanner und einem Operateur angeordnet werden kann, während der Patient vom Scanner bestrahlt wird. Der Strahlenschild ist aus einem Strahlung dämpfenden optisch transparenten Material wie beispielsweise einem bleihaltigen Kunststoff hergestellt. Dadurch kann der Operateur den Scanner und den Patienten sehen, während der Strahlenschild zwischen ihm und dem Scanner angeordnet ist und er durch den Strahlenschild geschützt ist. Jedoch kann der Operateur nicht auf den Patienten zugreifen, wenn er durch den Strahlenschild geschützt ist, so dass er für eine Intervention der oben beschriebenen Art, den Strahlenschild öffnen muss und dadurch nicht mehr vor der Strahlung des Scanners geschützt ist.

DE 10 2004 039 411 A1 betrifft eine Röntgenvorrichtung mit mitgeführtem Strahlenschutz, wobei der Streustrahlenschutz mittels einer Koppeleinheit in einer festen relativen Position bezüglich eines Bildempfängers und/oder eines Röntgenstrahlers als Referenzobjekts derart fixierbar ist, dass bei Verstellung des Referenzobjekts bezüglich der Patientenlagerung der Streustrahlenschutz mit dem Referenzobjekt mitgeführt wird.

DE 203 07 606 U1 betrifft eine Strahlenschutzvorrichtung zur Abschirmung einer von einer Strahlungsquelle abgestrahlten Strahlung, wobei die Strahlenschutzvorrichtung eine die Strahlung absorbierende transparente Strahlenschutzscheibe umfasst, welche an einer Standvorrichtung fixiert ist, wobei die Strahlenschutzscheibe gewölbt ausgeführt ist und in stehender Körperhaltung eines Benutzers körpernah führbar ist.

Der vorliegenden Erfindung liegt daher die Aufgabe zu Grunde, eine Strahlenschutzvorrichtung vorzuschlagen, die einen Operateur auch während einer Intervention schützen kann.

### Darstellung der Erfindung

Die Aufgabe wird erfindungsgemäß durch eine Strahlenschutzvorrichtung gelöst, wie sie im unabhängigen Patentanspruch 1 definiert ist. Vorteilhafte Ausführungsvarianten der erfindungsgemäßen Strahlenschutzvorrichtung ergeben sich aus den abhängigen Patentansprüchen.

Das Wesen der Erfindung besteht im Folgenden: Eine Strahlenschutzvorrichtung zur Abschirmung einer von einer Strahlungsquelle abgestrahlten Strahlung, insbesondere einer Röntgenstrahlung, umfasst eine erste Strahlenschutzscheibe, die mindestens eine Ausnehmung aufweist, und eine zweite Strahlenschutzscheibe, die relativ zu der ersten Strahlenschutzscheibe derart verstellbar ist, dass mindestens eine Ausnehmung mit der zweiten Strahlenschutzscheibe abdeckbar ist. Unter Strahlungsquelle wird in diesem Zusammenhang beispielsweise die Strahlenquelle eines Detektors beziehungsweise Scanners eines tomografischen Verfahrens verstanden und insbesondere die Röntgenstrahlenquelle eines Computertomografie-Scanners (CT-Scanner). Strahlenschutzscheibe bezieht sich in diesem Zusammenhang auf ein beispielsweise im Wesentlichen ebenes Bauteil, das die abgestrahlte Strahlung dämpft und somit die Umgebung auf seiner einen Seite vor der auf seiner anderen Seite vorhandenen Strahlung schützt. Die zweite Strahlenschutzscheibe kann zur ersten Strahlenschutzscheibe verstellbar sein, indem sie im Wesentlichen linear verschiebbar ist, wobei die Flächen der ersten und zweiten Strahlenschutzscheibe vorzugsweise parallel zueinander angeordnet sind. Alternativ ist die zweite Strahlenschutzscheibe im Wesentlichen drehbar gegenüber der ersten Strahlenschutzscheibe, wobei die Flächen der ersten und zweiten Strahlenschutzscheibe vorzugsweise parallel zueinander angeordnet sind. Die mindestens eine Ausnehmung kann sich beispielsweise von einem Rand der ersten Strahlenschutzscheibe in die Strahlenschutzscheibe hinein erstrecken. Die Ausnehmung kann alternativ als durchgehende Öffnung beziehungsweise Loch ausgestaltet sein. Insbesondere kann die Ausnehmung so dimensioniert sein, dass ein Operateur einen oder mehrere Finger, eine oder beide Hände oder einen oder beide Arme hindurch strecken kann.

In einem tomografischen Verfahren kann die erfindungsgemäße Strahlenschutzvorrichtung auf verschiedene Weise zwischen einem Operateur und einem Scanner angeordnet werden und den Operateur somit vor der abgegebenen Strahlung des Scanners schützen. Insbesondere kann sie vor einer Öffnung eines Tunnels des Scanners, in dem ein darzustellendes Objekt wie ein Patient oder ein bestimmter Teil seines Körpers einführbar ist, angeordnet werden. Die Öffnung des Tunnels kann dadurch in Richtung des Operateurs im Wesentlichen abgedeckt werden. Gleichzeitig ermöglicht das wahlweise Öffnen und Schließen der mindestens einen Ausnehmung, dass der Operateur durch die geöffnete mindestens eine Ausnehmung hindurch greifen kann, während er durch die Strahlenschutzvorrichtung vor der Strahlungsquelle geschützt ist. Dadurch ermöglicht die Strahlenschutzvorrichtung, dass beispielsweise eine Intervention am darzustellenden Objekt vollzogen werden kann und dass der Operateur gleichzeitig durch die Strahlenschutzvorrichtung geschützt ist. Ist die Intervention zumindest zwischenzeitlich beendet, so kann die mindestens eine Ausnehmung durch die zweite Strahlenschutzscheibe wieder abgedeckt werden. Damit wird ermöglicht, dass der Operateur jeweils verhältnismäßig gut vor der abgegebenen Strahlung geschützt ist und trotzdem ein Zugriff auf das Objekt jederzeit möglich ist.

Vorzugsweise ist die zweite Strahlenschutzscheibe derart ausgestaltet und relativ zu der ersten Strahlenschutzscheibe verstellbar, dass mit der zweiten Strahlenschutzscheibe wahlweise eine oder zwei oder mehrere an der ersten Strahlenschutzscheibe vorhandene Ausnehmungen abdeckbar ist/sind. Eine solche Ausgestaltung der zweiten Strahlenschutzscheibe ermöglicht, dass mehrere Ausnehmungen in der ersten Strahlenschutzscheibe wahlweise zugänglich sind und dass die Strahlenschutzvorrichtung trotzdem nur verhältnismäßig wenige Bauteile umfasst. Damit kann die Strahlenschutzvorrichtung verhältnismäßig einfach, kostengünstig und robust ausgestaltet sein.

Alternativ dazu weist die erste Strahlenschutzscheibe mindestens zwei Ausnehmungen auf und mindestens zwei zweite Strahlenschutzscheiben sind vorgesehen, die relativ zu der ersten Strahlenschutzscheibe derart verstellbar sind, dass mit jeweils einer zweiten Strahlenschutzscheibe eine der mindestens zwei Ausnehmungen abdeckbar ist. Eine solche Ausgestaltung ermöglicht, dass ebenfalls mehrere Ausnehmungen in der ersten Strahlenschutzscheibe wahlweise zugänglich sind und dass die Strahlenschutzvorrichtung verhältnismäßig einfach zu bedienen ist. Die Strahlenschutzvorrichtung weist eine Drehbefestigung auf zum relativen Verstellen der mindestens einen zweiten Strahlenschutzscheibe gegenüber der ersten Strahlenschutzscheibe um eine Drehachse, wobei mehrere zweite Strahlenschutzscheiben vorzugsweise um eine gemeinsame Drehachse verstellbar sind. Eine solche Drehbefestigung ermöglicht eine verhältnismäßig einfache robuste Konstruktion der Strahlenschutzvorrichtung.

Vorzugsweise umfasst die Strahlenschutzvorrichtung eine Verschiebeeinrichtung, mit der mindestens eine der zweiten Strahlenschutzscheiben relativ zur ersten Strahlenschutzscheibe verschiebbar ist. Eine solche Verschiebeeinrichtung kann beispielsweise zum linearen Verschieben der mindestens einen der zweiten Strahlenschutzscheiben ausgestaltet sein und ermöglicht ein verhältnismäßig einfaches Öffnen und Schließen der mindestens einen Ausnehmung der ersten Strahlenschutzscheibe.

Gemäß einem weiteren Aspekt der Erfindung weist die erste Strahlenschutzscheibe eine rechteckförmige Fläche auf, wobei vorzugsweise die Ecken der rechteckförmigen Fläche abgerundet sind, und wobei besonders bevorzugt ein erstes Paar einander diagonal gegenüberliegende Ecken eine erste Abrundung aufweisen und ein zweites Paar einander diagonal gegenüberliegende Ecken eine weitere Abrundung aufweisen, wobei die erste Abrundung vorzugsweise einen größeren Radius aufweist als die weitere Abrundung. Vorzugsweise weist das zweite Paar Ecken eine zweite und eine dritte Abrundung auf, wobei die zweite Abrundung einen größeren Radius aufweist als die dritte Abrundung. Eine so geformte erste Strahlenschutzscheibe ermöglicht, dass einerseits keine scharfkantigen Ecken vorhanden sind und dass andererseits der Platzbedarf bei einer Rotation der ersten Strahlenschutzscheibe verhältnismäßig gering ist. Zudem kann die Ausgestaltung der Ecken mit der ersten Abrundung entsprechend der Krümmung einer Öffnung eines Tunnels eines Scanners ausgestaltet sein, so dass sie diese Öffnung vorzugsweise abdecken kann und gegebenenfalls mindestens teilweise in die Öffnung einführbar ist. Typische Durchmesser von solchen Tunnels betragen etwa 70 cm oder etwa 80 cm, d.h der entsprechende Radius beträgt etwa 35 cm oder etwa 40 cm. Der Radius der ersten Abrundung ist vorzugsweise gleich oder kleiner als der Radius eines Tunnels. Damit kann ermöglicht werden, dass die Strahlenschutzvorrichtung während der Bestrahlung variabel am Objekt beziehungsweise am Patienten platziert werden kann und somit verhältnismäßig einfach interveniert werden kann. Die zweite Strahlenschutzscheibe weist vorzugsweise eine rechteckförmige Fläche auf, die kleiner als die rechteckförmige Fläche der ersten Strahlenschutzscheibe ist, wobei vorzugsweise eine Seite der Fläche mindestens teilweise schräg verläuft und wobei die Fläche und Form der zweiten Strahlenschutzscheibe an die jeweils abzudeckende Ausnehmung in der ersten Strahlenschutzscheibe angepasst ist. Vorzugsweise ist die zweite Strahlenschutzscheibe so ausgebildet und an der ersten Strahlenschutzscheibe angeordnet, dass sie an einer Seite mit dem Rand der ersten Strahlenschutzscheibe einen durchgehenden Außenrand bildet und an ihren weiteren Seiten über den Rand der abzudeckenden Ausnehmung hinausragt, wenn die zweite Strahlenschutzscheibe die Ausnehmung verschließt. Mit der mindestens teilweise abgeschrägten Ausgestaltung der zweiten Strahlenschutzscheibe kann Material bei der Herstellung der zweiten Strahlenschutzscheibe eingespart werden, ohne dass deren Abdeckeigenschaften beeinträchtigt werden. Mit einer solchen Ausgestaltung der zweiten Strahlenschutzscheibe kann eine bevorzugte Bedienbarkeit und eine effektive Abschirmwirkung erreicht werden.Die Strahlenschutzvorrichtung umfasst ein Anschlusselement zur Anbringung an einen Halteteil, wobei das Halteteil vorzugsweise an einer feststehenden Einrichtung in einem Raum oder an einem Untersuchungsgerät oder an einem Gestell eines Untersuchungstisches befestigbar ist. Ein solches Halteteil ermöglicht eine stabile Befestigung der Strahlenschutzvorrichtung unter Ermöglichung einer flexiblen Platzierung mit bevorzugten Bewegungsfreiheitsgraden. Dabei ist die zweite Strahlenschutzscheibe vorzugsweise an dem Anschlusselement drehbar befestigt ist.

Gemäß einem weiteren Aspekt der Erfindung bestehen die erste Strahlenschutzscheibe und die zweite Strahlenschutzscheibe aus einem transparenten Material, vorzugsweise aus Bleiacrylglas. Eine solche transparente Ausgestaltung der Strahlenschutzvorrichtung ermöglicht, dass ein Operateur das Objekt während der Bestrahlung geschützt visuell kontrollieren kann und insbesondere Interventionen daran vornehmen kann.

### Kurze Beschreibung der Zeichnungen

Im Folgenden wird die erfindungsgemäße Strahlenschutzvorrichtung unter Bezugnahme auf die beigefügten Zeichnungen anhand von Ausführungsbeispielen detaillierter beschrieben. Es zeigen:
Fig. 1 eine schematische Aufsicht einer ersten Strahlenschutzscheibe eines ersten Ausführungsbeispiels einer erfindungsgemäßen Strahlenschutzvorrichtung;
Fig. 2 eine schematische Aufsicht einer zweiten Strahlenschutzscheibe der Strahlenschutzvorrichtung von Fig. 1;
Fig. 3 eine schematische Aufsicht der Strahlenschutzvorrichtung von Fig. 1 und von Fig. 2 mit der zweiten Strahlenschutzscheibe auf der ersten Strahlenschutzscheibe angeordnet;
Fig. 4 eine schematische Ansicht eines Halteteils und der über ein Kugelgelenk daran befestigten ersten Strahlenschutzscheibe der Strahlenschutzvorrichtung von Fig. 1;
Fig. 5 eine schematische Aufsicht einer ersten Strahlenschutzscheibe eines zweiten Ausführungsbeispiels einer erfindungsgemäßen Strahlenschutzvorrichtung;
Fig. 6 eine schematische Aufsicht eines dritten Ausführungsbeispiels einer erfindungsgemäßen an einem Halteteil befestigten Strahlenschutzvorrichtung;
Fig. 7 eine schematische Seitenansicht der Strahlenschutzvorrichtung von Fig. 6; und
Fig. 8 eine perspektivische Ansicht der Strahlenschutzvorrichtung von Fig. 6.

### Ausführungsformen der Erfindung

Bestimmte Ausdrücke werden in der folgenden Beschreibung aus praktischen Gründen verwendet und sind nicht einschränkend zu verstehen. Die Wörter "rechts", "links", "unten" und "oben" bezeichnen Richtungen in der Zeichnung, auf die Bezug genommen wird. Die Ausdrücke "nach innen" und "nach außen" bezeichnen Richtungen hin zum oder weg vom geometrischen Mittelpunkt der Strahlenschutzvorrichtung sowie benannter Teile derselben. Die Terminologie umfasst die oben ausdrücklich erwähnten Wörter, Ableitungen von denselben und Wörter ähnlicher Bedeutung.

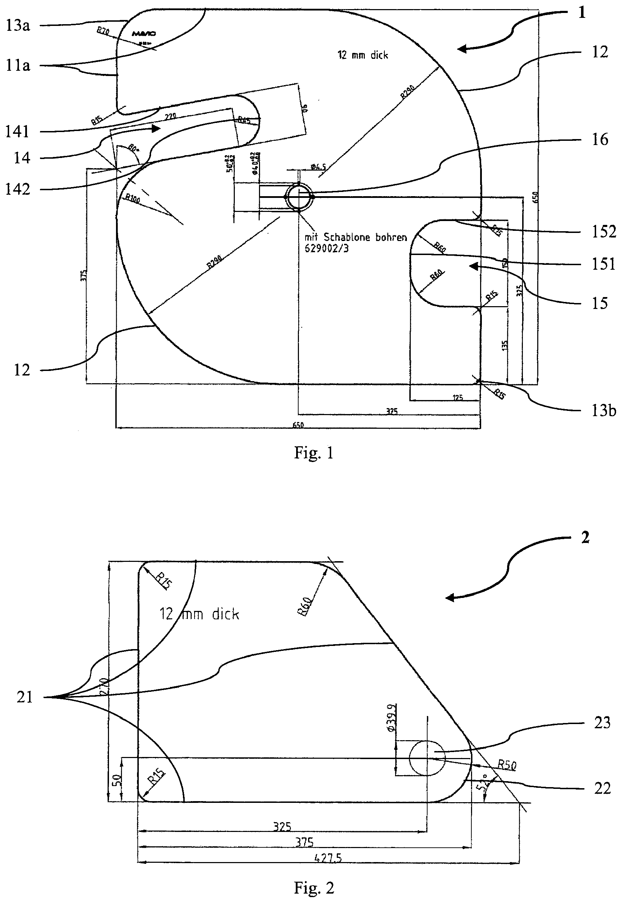

In Fig. 1 ist eine erste Strahlenschutzscheibe 1 eines ersten Ausführungsbeispiels einer erfindungsgemäßen Strahlenschutzvorrichtung gezeigt. Die erste Strahlenschutzscheibe 1 ist als eine im Wesentlichen rechteckige beziehungsweise quadratische Platte von beispielsweise etwa 12 mm Dicke aus Bleiacrylglas hergestellt, wobei die vier Ecken jeweils abgerundet sind. Sie weist vier Seiten 11 auf, die ohne abgerundete Ecken eine Länge von beispielsweise etwa 650 mm Länge aufweisen. Ein erstes Paar einander diagonal gegenüberliegende Ecken der vier Ecken weisen eine erste Abrundung 12 auf und ein zweites Paar einander gegenüberliegende Ecken der vier Ecken zweite und dritte Abrundungen 13a und 13 b. Die erste Abrundung 12 weist einen größeren Radius von beispielsweise etwa 290 mm auf als die zweite Abrundung 13a, die einen Radius von beispielsweise etwa 70 mm aufweist, und als die dritte Abrundung 13b, die einen Radius von etwa 15 mm aufweist.

An einer seiner Seiten 11 beginnend (in Figur 1 oben links) ist eine erste Ausnehmung 14 an der ersten Strahlenschutzscheibe 1 nach innen ausgestaltet und an einer dieser Seite 11 gegenüberliegenden Seite 11 beginnend (in Figur 1 unten rechts) eine zweite Ausnehmung 15. Die erste Ausnehmung 14 ist im Wesentlichen rechteckig mit Seiten 141 von einer Länge von beispielsweise etwa 220 mm und einer Breite von beispielsweise etwa 90 mm, wobei die Seiten 141 gegen ihr inneres Ende über einen Bogen 142 mit einem Radius von beispielsweise etwa 45 mm miteinander verbunden sind. Die zweite Ausnehmung 15 ist ebenfalls im Wesentlichen rechteckig mit Seiten 151 mit einer Länge von beispielsweise etwa 150 mm und einem Bogen 152 mit einer Länge von beispielsweise etwa 125 mm ausgestaltet. Die Übergänge von der Seite 151 zu dem Bogen 152 sind jeweils abgerundet ausgestaltet.

Zentral umfasst die erste Strahlenschutzscheibe 1 eine durchgehende abgestufte Bohrung 16 mit einem ersten Durchmesser von beispielsweise etwa 50 mm und einem zweiten Durchmesser von beispielsweise etwa 40 mm. Die Übergänge von den Seiten 11 der ersten Strahlenschutzscheibe 1 zu der ersten Ausnehmung 14 und zu der zweiten Ausnehmung 15 sind jeweils abgerundet ausgestaltet.

Die erste Ausnehmung 14 und die zweite Ausnehmung 15 sind so dimensioniert, dass beispielsweise jeweils ein menschlicher Arm hindurch ragen kann. Vorzugsweise ist die erste Ausnehmung an den Durchmesser eines Unterarmes eines Operateurs und die zweite Ausnehmung an den Durchmesser eines Oberarmes eines Operateurs angepasste. Im Betrieb kann die erste Strahlenschutzscheibe 1 bevorzugt vor einer Öffnung eines Tunnels eines Scanners für beispielsweise eine Computertomografie angeordnet werden. Dabei ermöglicht die auf die Geometrie des Tunnels, der typischerweise einen Durchmesser von etwa 700 mm oder von etwa 800 mm aufweist, insbesondere bezüglich dessen Breite und Krümmungsradius angepasste Ausgestaltung der ersten Strahlenschutzscheibe 1 ein bevorzugtes Anordnen der Strahlenschutzscheibe 1 in verschiedenen Lagen vor der Öffnung des Tunnels und auch zumindest teilweise im Innern des Tunnels. Die Ausgestaltung der ersten Strahlenschutzscheibe 1 kann also einem Operateur ermöglichen, dass dieser angepasst auf eine vorgesehene Intervention die erste Strahlenschutzscheibe 1 so positioniert, dass er einerseits bequem und zielgenau durch die Ausnehmungen durchgreifen kann und dass er andererseits durch die erste Strahlenschutzscheibe 1 von einer Strahlenquelle des Scanners geschützt ist.

Für die gesamte weitere Beschreibung gilt folgende Festlegung. Sind in einer Figur zum Zweck zeichnerischer Eindeutigkeit Bezugszeichen enthalten, aber im unmittelbar zugehörigen Beschreibungstext nicht erwähnt, so wird auf deren Erläuterung in vorangehenden Figurenbeschreibungen Bezug genommen. Sind außerdem im unmittelbar zu einer Figur gehörigen Beschreibungstext Bezugszeichen erwähnt, die in der zugehörigen Figur nicht enthalten sind, so wird auf die vorangehenden Figuren verwiesen.

Fig. 2 zeigt eine zweite Strahlenschutzscheibe 2 des ersten Ausführungsbeispiels einer erfindungsgemäßen Strahlenschutzvorrichtung. Die zweite Strahlenschutzscheibe 2 ist im Wesentlichen als ein plattenförmiges Rechteck mit einer teilweise schrägen Längsseite, also als ein plattenförmiges rechtwinkliges Trapez, von beispielsweise etwa 12 mm Dicke aus Bleiacrylglas hergestellt, wobei die vier Ecken jeweils abgerundet sind. Sie weist vier Seiten 21 auf und hat eine Höhe von beispielsweise etwa 270 mm sowie eine Länge von beispielsweise etwa 375 mm. Im Bereich des spitzen Winkels der zweiten Strahlenschutzscheibe 2, der eine Abrundung 22 mit einem Radius von beispielsweise etwa 50 mm aufweist, umfasst die zweite Strahlenschutzscheibe 2 eine durchgehende Bohrung 23 von beispielsweise etwa 39.9 mm Durchmesser.

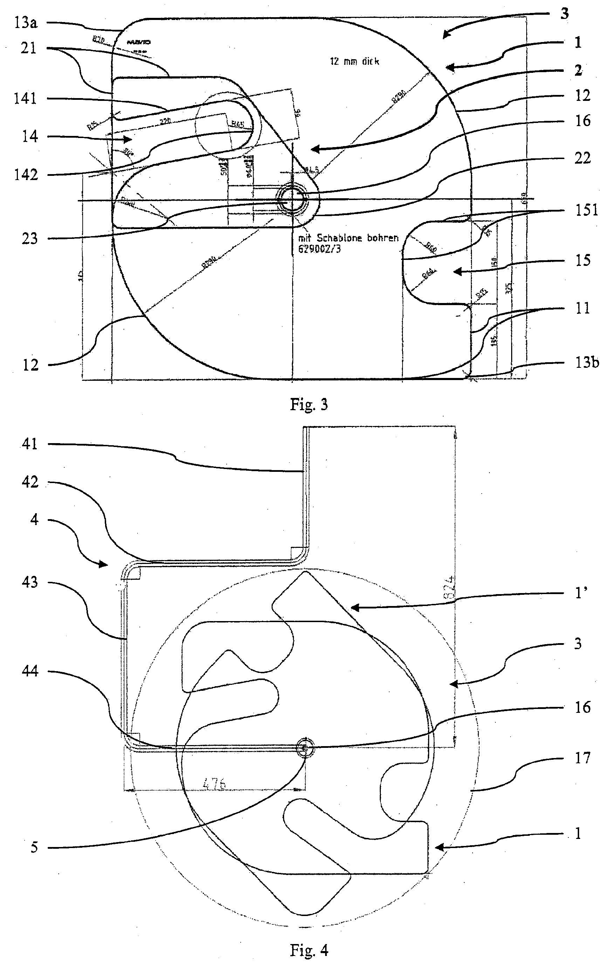

In Fig. 3 sind die erste Strahlenschutzscheibe 1 und die zweite Strahlenschutzscheibe 2 der Strahlenschutzvorrichtung 3 aufeinander gezeigt. Die abgestufte Bohrung 16 der ersten Strahlenschutzscheibe 1 liegt dabei anliegend an der Bohrung 23 der zweiten Strahlenschutzscheibe 2, so dass sie zusammen eine gemeinsame Rotationsachse beziehungsweise gemeinsame Drehachse definieren. In der in Fig. 3 dargestellten Stellung ist die zweite Strahlenschutzscheibe 2 so verstellt beziehungsweise gedreht, dass sie die erste Ausnehmung 14 der ersten Strahlenschutzscheibe 1 vollständig abdeckt und diese damit nicht zugänglich ist. Die zweite Ausnehmung 15 der ersten Strahlenschutzscheibe 1 ist in dieser Stellung hingegen nicht von der zweiten Strahlenschutzscheibe 2 abgedeckt und vollständig zugänglich.

Im Betrieb kann die zweite Strahlenschutzscheibe 2 relativ zur ersten Strahlenschutzscheibe 1 verstellt werden, indem sie um die Rotationsachse gedreht wird. Dabei kann sie wahlweise so angeordnet sein, dass sie entweder die erste Ausnehmung 14 der ersten Strahlenschutzscheibe 1, die zweite Ausnehmung 15 der ersten Strahlenschutzscheibe 1 oder keine Ausnehmung der ersten Strahlenschutzscheibe 1 abdeckt. Der Operateur kann also je nach Bedarf wahlweise eine der beiden oder auch beide Ausnehmungen 14, 15 der ersten Strahlenschutzscheibe 1 geöffnet halten, damit er durch die Ausnehmungen an der Strahlenschutzvorrichtung 3 beispielsweise zur Intervention am zu scannenden Objekt greifen kann.

Fig. 4 zeigt die Strahlenschutzvorrichtung 3, die über ein Kugelgelenk 5 als Anschlussmittel und Drehbefestigung drehbar und schwenkbar an einem Halteteil 4 befestigt ist. Die zweite Strahlenschutzscheibe 2 der Strahlenschutzvorrichtung ist in Fig. 4 nicht dargestellt. Das Halteteil 4 weist einen ersten Abschnitt 41 auf, der beispielsweise an einem Tragarm befestigt sein kann und beispielsweise darüber an einer Decke eines Raumes, an einer feststehenden Einrichtung im Raum, an einem Untersuchungsgerät oder an einem Gestell eines Untersuchungstisches montiert sein kann. Der erste Abschnitt 41 geht über einen gebogenen rechten Winkel in einen zweiten Abschnitt 42 über, der rechtwinklig zum ersten Abschnitt 41 angeordnet ist. Der zweite Abschnitt 42 geht ebenfalls über einen gebogenen rechten Winkel in einen dritten Abschnitt 43 über, der wiederum über einen gebogenen rechten Winkel in einen vierten Abschnitt 44 übergeht. Somit sind der erste Abschnitt 41 und der dritte Abschnitt 43 sowie der zweite Abschnitt 42 und der vierte Abschnitt 44 jeweils parallel zueinander angeordnet. Gleichzeitig sind der zweite Abschnitt 42 und der vierte Abschnitt 44 rechtwinklig zum ersten Abschnitt 41 und zum dritten Abschnitt 43 angeordnet. Der zweite Abschnitt 42, der dritte Abschnitt 43 und der vierte Abschnitt 44 weisen eine Länge von beispielsweise etwa 475 mm auf.

An seinem dem dritten Abschnitt 43 abgewandten Längsende ist der vierte Abschnitt 44 über das Kugelgelenk 5 mit der ersten Strahlenschutzscheibe 1 verbunden. Das Kugelgelenk weist einen Bolzen auf, der durch die Bohrung 16 der ersten Strahlenschutzscheibe 1 ragt. Die erste Strahlenschutzscheibe ist dabei vorzugsweise fest an dem Bolzen befestigt und kann mittels des Kugelgelenks in eine beliebige Dreh- und Schwenkstellung gebracht werden. Die erste Strahlenschutzscheibe kann alternativ um die durch die Bohrung 16 definierte Rotationsachse bzw. den hindurchgehenden Bolzen des Kugelgelenks in der Ebene seiner Oberfläche drehbar befestigt sein. Zur Illustration ist die Strahlenschutzscheibe 1 in Fig. 4 in zwei Rotationsstellungen dargestellt. Die Darstellung der Strahlenschutzscheibe 1 ist dabei so gedreht, dass die erste Ausnehmung 14 an einer linken Seite 11 liegt und dass die zweite Ausnehmung 15 an einer rechten Seite 11 liegt. Die vier Seiten 11 sind dabei im Wesentlichen horizontal oben und unten sowie vertikal links und rechts angeordnet. Die Darstellung der Strahlenschutzscheibe 1' ist so gedreht, dass die vier Seiten 11 im Wesentlichen mit den gegenüberliegenden Ecken nach oben und unten bzw. links und rechts angeordnet sind. Dabei liegt die erste Ausnehmung 14 an einer rechten unteren Seite 11 und die zweite Ausnehmung 15 an einer linken oberen Seite 11. Die Ecken der ersten Strahlenschutzscheibe 1 beschreiben einen Rotationsumfang 17, der den maximalen Platzbedarf der ersten Strahlenschutzscheibe 1 sowie die minimale Länge des dritten Abschnitts 43 und des vierten Abschnitts 44 des Halteteils 4 festlegt. Durch die abgerundete Ausgestaltung der Ecken der ersten Strahlenschutzscheibe 1 kann der Rotationsumfang 17 verhältnismäßig klein gehalten sein.

An dem Bolzen des Kugelgelenks 5 ist vorzugsweise außerdem die zweite Strahlenschutzscheibe 2 drehbar befestigt. Dabei ragt der Bolzen durch die Bohrung 23 in der zweiten Strahlenschutzscheibe 2. Der Bolzen weist Abschnitte mit unterschiedlichem Durchmesser auf, die jeweils den Durchmessern der Bohrung 16 in der ersten Strahlenschutzscheibe 1 und der Bohrung 23 in der zweiten Strahlenschutzscheibe 2 entsprechen. Die Länge der Abschnitte des Bolzens entspricht im Wesentlichen der jeweiligen Dicke der ersten bzw. zweiten Strahlenschutzscheibe. Vorzugsweise ist der Abstand der beiden Strahlenschutzscheiben in Dickenrichtung, d.h. senkrecht zu ihren Flächen klein und liegt bevorzugt in einem Bereich von 0,1mm bis 1mm.

Das Halteteil 4 kann bevorzugt um den ersten Abschnitt 41 drehbar montiert sein. Zudem kann die erste Strahlenschutzscheibe 1 um den vierten Abschnitt 44 des Halteteils 4 kippbar angeordnet sein. Durch diese Ausgestaltung des Halteteils 4 und der ersten Strahlenschutzscheibe 1 kann die Strahlenschutzvorrichtung 3 vom Operateur variabel in einer geeigneten Position angeordnet werden.

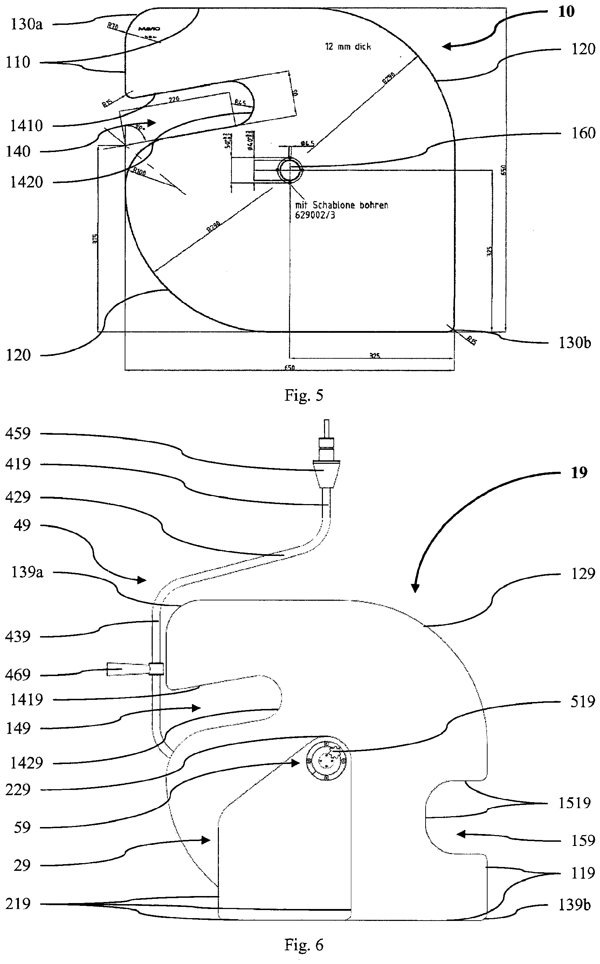

In Fig. 5 ist eine erste Strahlenschutzscheibe 10 eines zweiten Ausführungsbeispiels einer erfindungsgemäßen Strahlenschutzvorrichtung gezeigt. Die erste Strahlenschutzscheibe 10 ist dabei ähnlich zur ersten Strahlenschutzscheibe 1 der Fig. 1 bis Fig. 4 ausgestaltet, wobei diese nur eine Ausnehmung aufweist. Insbesondere umfasst die Strahlenschutzscheibe 10 vier Seiten 110, zwei gegenüberliegende Ecken mit einer Abrundung 120 von größerem Radius als zwei andere gegenüberliegende Ecken mit Abrundungen 130 von kleinerem Radius und eine zentrale abgestufte Bohrung 160. Des Weiteren erstreckt sich die erste Ausnehmung 160 von der linken Seite 110 der Strahlenschutzscheibe 10 nach innen. Die Ausnehmung weist zwei gegenüberliegende im Wesentlichen parallele Seiten 1410 und einen Bogen 1420 auf, der beide Seiten verbindet und das Ende der Ausnehmung bildet. Der Abstand zwischen beiden Seiten ist vorzugsweise an den Durchmesser eines Unterarmes eines Operateurs angepasst. Das Verhältnis der Länge der Ausnehmung zu dem Abstand zwischen beiden Seiten liegt vorzugsweise in einem Bereich von 1:1 bis 3:1.

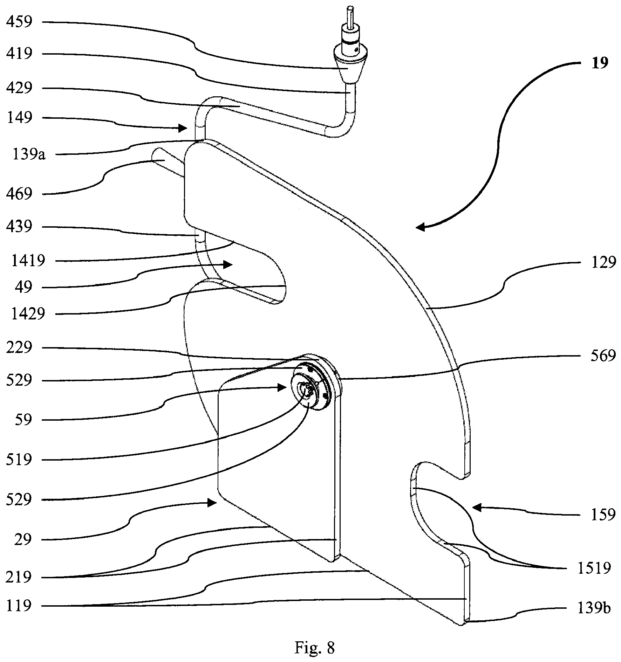

Fig. 6, Fig. 7 und Fig. 8 zeigen ein drittes Ausführungsbeispiel einer erfindungsgemäßen Strahlenschutzvorrichtung, die an einem Halteteil 49 befestigt ist. Die Strahlenschutzvorrichtung umfasst eine erste Strahlenschutzscheibe 19, die strukturell im Wesentlichen der oben beschriebenen ersten Strahlenschutzscheibe 1 des ersten Ausführungsbeispiels einer erfindungsgemäßen Strahlenschutzvorrichtung entspricht, und eine zweite Strahlenschutzscheibe 29, die strukturell im Wesentlichen der oben beschriebenen zweiten Strahlenschutzscheibe 2 des ersten Ausführungsbeispiels einer erfindungsgemäßen Strahlenschutzvorrichtung entspricht. Demgemäß ist die erste Strahlenschutzscheibe 19 als eine im Wesentlichen rechteckige beziehungsweise quadratische Platte mit vier Seiten 119 hergestellt, wobei die vier Ecken jeweils abgerundet sind. Ein erstes Paar einander diagonal gegenüberliegende Ecken der vier Ecken weisen eine erste Abrundung 129 auf und ein zweites Paar einander gegenüberliegende Ecken der vier Ecken zweite und dritte Abrundungen 139a und 139b. Die erste Abrundung 129 weist einen größeren Radius auf als die zweite Abrundung 139a und als die dritte Abrundung 139b. An einer ihrer Seiten 119 beginnend (in Fig. 6 links) ist eine erste Ausnehmung 149 an der ersten Strahlenschutzscheibe 19 nach innen ausgestaltet und an einer dieser Seite 119 gegenüberliegenden Seite 119 beginnend (in Fig. 6 rechts) eine zweite Ausnehmung 159. Die erste Ausnehmung 149 ist im Wesentlichen rechteckig mit Längsseiten 1419 ausgestaltet, wobei die Seiten 1419 gegen ihr inneres Ende über einen Bogen 1429 miteinander verbunden sind. Die zweite Ausnehmung 159 ist ebenfalls im Wesentlichen rechteckig mit Seiten 1519 ausgestaltet. Die Übergänge der Seiten 1519 sind jeweils abgerundet ausgestaltet. Die Übergänge von den Seiten 119 der ersten Strahlenschutzscheibe 19 zu der ersten Ausnehmung 149 und zu der zweiten Ausnehmung 159 sind jeweils abgerundet ausgestaltet.

Die zweite Strahlenschutzscheibe 29 ist im Wesentlichen als ein plattenförmiges Rechteck mit einer teilweise schrägen Längsseite, also als ein plattenförmiges rechtwinkliges Trapez, mit vier Seiten 219 hergestellt, wobei die vier Ecken jeweils abgerundet sind. Im Bereich des spitzen Winkels der zweiten Strahlenschutzscheibe 29, der eine Abrundung 229 aufweist, ist die zweite Strahlenschutzscheibe 29 über eine Befestigungsstruktur 59 mit einer Rändelschraube 519 drehbar mit der ersten Strahlenschutzscheibe 19 verbunden. Über die Befestigungsstruktur 59 sind die erste Strahlenschutzscheibe 19 und die zweite Strahlenschutzscheibe 29 gleichzeitig auch an einem Halteteil 49 befestigt. Das Halteteil 49 umfasst einen Adapter 459 über den es an einem Tragarm montiert werden kann. Es weist einen an den Adapter 459 anschließenden ersten Abschnitt 419 auf, der über einen gebogenen stumpfen Winkel in einen geneigten zweiten Abschnitt 429 übergeht. Der zweite Abschnitt 429 geht ebenfalls über einen gebogenen stumpfen Winkel in einen dritten Abschnitt 439 über, der wiederum über einen gebogenen stumpfen Winkel in einen vierten Abschnitt 449 übergeht. Am dritten Abschnitt 439 des Halteteils 49 ist ein Betätigungsgriff 469 angebracht, über den die Strahlenschutzvorrichtung 19 durch den Operateur positioniert und eingestellt werden kann.

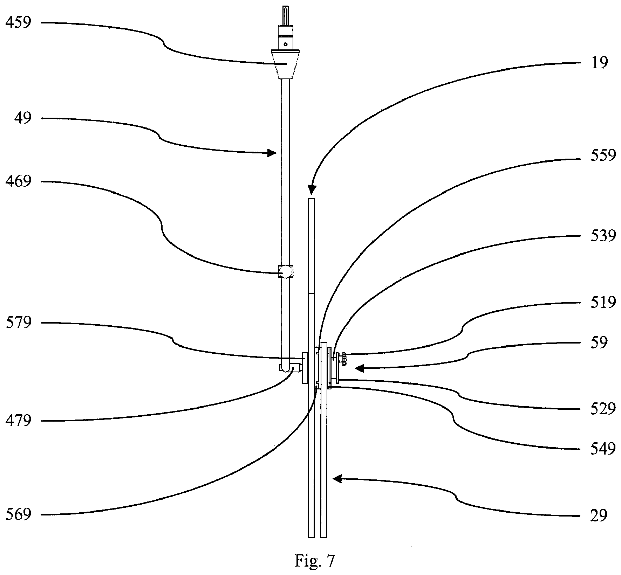

In Fig. 7 ist die Strahlenschutzvorrichtung mit seiner ersten Strahlenschutzscheibe 19 und seiner zweiten Strahlenschutzscheibe 29 von der Seite gezeigt, wobei unter anderem die Befestigungsstruktur 59 detaillierter ersichtlich ist. Die Befestigungsstruktur 59 umfasst einen zylindrischen Kolben 539, der die durchgehende Bohrung der zweiten Strahlenschutzscheibe 29 und die durchgehende Bohrung der ersten Strahlenschutzscheibe 19 durchragt. Auf einer dem Halteteil 49 abgewandten axialen Längsende des Kolbens 539 (in Fig. 7 rechts) schließt ein Flanschabschnitt 529 an den Kolben 539 an, der den Kolben 539 in radialer Richtung nach außen überragt. Die Befestigungsstruktur 59 umfasst weiter eine erste, eine zweite, eine dritte und eine vierte vom Kolben 539 durchragte Ringscheibe 549, 559, 569 und 579. Zwischen der ersten Ringscheibe 549 und der zweiten Ringscheibe 559 ist die zweite Strahlenschutzscheibe 29 angeordnet und ortsfest mit diesen beiden verbunden. Analog dazu ist zwischen der dritten Ringscheibe 569 und der vierten Ringscheibe 579 die erste Strahlenschutzscheibe 19 angeordnet und ortsfest mit diesen verbunden.

Die Befestigungsstruktur 59 ist an einem Montageabschnitt 479 des Halteteils 49 montiert. In der in Fig. 7 gezeigten Stellung drückt die Rändelschraube 519 die erste Ringscheibe 549 zusammen mit der zweiten Strahlenschutzscheibe und der zweiten Ringscheibe 559 an die erste Strahlenschutzscheibe 19, so dass die erste Strahlenschutzscheibe 19 und die zweite Strahlenschutzscheibe 29 drehfest miteinander verbunden sind.

Zur Vorbestimmung möglicher Drehpositionen der zweiten Strahlenschutzscheibe 29 bezüglich der ersten Strahlenschutzscheibe 19, weist die zweite Ringscheibe 559 Rastnasen auf, die sich in Richtung der dritten Ringscheibe 569 erstrecken. An der dritten Ringscheibe 569 sind entsprechende Aufnahmen ausgebildet, in die die Rastnasen der zweiten Ringscheibe 559 eingreifen. Zum Verdrehen der zweiten Strahlenschutzscheibe 29 in Bezug auf die erste Strahlenschutzscheibe 19 wird die Rändelschraube 519 gelöst und die zweite Strahlenschutzscheibe 29 auf dem Kolben 539 von der ersten Strahlenschutzscheibe 19 weg bewegt, bis die Rastnasen der zweiten Ringscheibe 559 aus den Aufnahmen der dritten Ringscheibe 569 entfernt sind. Dabei verhindert der durch den Flanschabschnitt 529 der Befestigungsstruktur 59 gebildete Anschlag, dass die zweite Strahlenschutzscheibe 29 unbeabsichtigt demontiert werden kann. Die gelöste zweite Strahlenschutzscheibe 29 kann dann so in verschiedene Positionen gedreht werden, dass sie wunschgemäß die erste Ausnehmung 149, die zweite Ausnehmung 159 oder keine Ausnehmung der ersten Strahlenschutzscheibe 19 abdeckt. In dieser Stellung besteht ein genügend großer Freiraum zwischen der großen und der kleinen Scheibe, sodass ein Anwender in den Freiraum mit der Hand hineingreifen kann und die Scheiben leichter gereinigt werden können. Ist die zweite Strahlenschutzscheibe 29 wunschgemäß verdreht, wird sie wieder auf dem Kolben 539 in Richtung der ersten Strahlenschutzscheibe 19 verschoben, bis die Rastnasen der zweiten Ringscheibe 559 in die Aufnahmen der dritten Ringscheibe 569 greifen. Durch festziehen der Rändelschraube 519 wird die erste Strahlenschutzscheibe 29 in dieser Stellung gehalten und drehfest mit der zweiten Strahlenschutzscheibe 19 verbunden.

Obwohl die Erfindung mittels der Figuren und der zugehörigen Beschreibung dargestellt und detailliert beschrieben ist, sind diese Darstellung und diese detaillierte Beschreibung illustrativ und beispielhaft zu verstehen und nicht als die Erfindung einschränkend. Insbesondere umfasst die Erfindung ebenfalls Ausführungsformen mit jeglicher Kombination von Merkmalen, die vorstehend oder nachfolgend zu verschiedenen Ausführungsformen genannt oder gezeigt sind. Zum Beispiel kann die Erfindung auch durch folgende weitere konstruktive Variationen realisiert sein:
- Die Strahlenschutzvorrichtung kann auch mehrere zweite Strahlenschutzscheiben umfassen. Insbesondere kann das in der Fig. 1 bis Fig. 4 gezeigte Ausführungsbeispiel der Strahlenschutzvorrichtung eine zweite zweite Strahlenschutzscheibe aufweisen, die der ersten zweiten Strahlenschutzscheibe entsprechen kann. Damit wird ermöglicht, dass beide Ausnehmungen der ersten Strahlenschutzscheibe gleichzeitig abgedeckt werden können.
- An den peripheren Enden der ersten Strahlenschutzscheibe können Verbindungsmittel wie beispielsweise Druckknöpfe vorgesehen sein, an denen nach Bedarf zusätzliche Strahlenschutzmittel angebracht werden können. Damit kann je nach Bedarf ein Spalt, der sich beispielsweise unterhalb der ersten Strahlenschutzscheibe befindet, beispielsweise mittels eines Schutzvorhangs einfach und flexibel abgedeckt werden.

Die Erfindung umfasst ebenfalls einzelne Merkmale in den Figuren auch wenn sie dort im Zusammenhang mit anderen Merkmalen gezeigt sind und/oder vorstehend oder nachfolgend nicht genannt sind Auch können einzelne von alternativen Ausführungsformen oder alternative Merkmale, die in den Figuren und der Beschreibung beschrieben sind, vom Erfindungsgegenstand ausgeschlossen sein.

Im Weiteren schließt der Ausdruck "umfassen" und Ableitungen davon andere Elemente oder Schritte nicht aus. Ebenfalls schließt der unbestimmte Artikel "ein" bzw. "eine" und Ableitungen davon eine Vielzahl nicht aus. Die Funktionen mehrerer in den Ansprüchen aufgeführter Merkmale können durch eine Einheit erfüllt sein. Die Begriffe "im Wesentlichen", "etwa", "ungefähr" und dergleichen in Verbindung mit einer Eigenschaft beziehungsweise einem Wert definieren insbesondere auch genau die Eigenschaft beziehungsweise genau den Wert. Alle Bezugszeichen in den Ansprüchen sind nicht als den Umfang der Ansprüche einschränkend zu verstehen.

## Patentansprüche

1. Strahlenschutzvorrichtung (3) zur Abschirmung einer von einer Strahlungsquelle abgestrahlten Strahlung, insbesondere einer Röntgenstrahlung, mit einer ersten Strahlenschutzscheibe (1; 10), wobei die erste Strahlenschutzscheibe (1; 10) mindestens eine Ausnehmung (14, 15; 140) aufweist, und mit mindestens einer zweiten Strahlenschutzscheibe (2), die relativ zu der ersten Strahlenschutzscheibe (1; 10) derart verstellbar ist, dass mindestens eine Ausnehmung (14, 15; 140) mit der zweiten Strahlenschutzscheibe (2) abdeckbar ist, und mit einem Anschlusselement (5) zur Anbringung an ein Halteteil (4), wobei das Halteteil (4) an einer feststehenden Einrichtung in einem Raum oder an einem Untersuchungsgerät oder an einem Gestell eines Untersuchungstisches befestigbar ist,
**dadurch gekennzeichnet, dass** die erste Strahlenschutzscheibe (1; 10) und die zweite Strahlenschutzscheibe (2) an dem Anschlusselement (5) befestigt sind und die zweite Strahlenschutzscheibe (2) an dem Anschlusselement (5) drehbar befestigt ist.

2. Strahlenschutzvorrichtung (3) nach Anspruch 1, wobei die zweite Strahlenschutzscheibe (2) derart ausgestaltet ist und relativ zu der ersten Strahlenschutzscheibe (1; 10) verstellbar ist, dass mit der zweiten Strahlenschutzscheibe (2) wahlweise eine oder zwei oder mehrere an der ersten Strahlenschutzscheibe (1; 10) vorhandene Ausnehmungen (14, 15; 140) abdeckbar ist/sind.

3. Strahlenschutzvorrichtung (3) nach Anspruch 1, wobei die erste Strahlenschutzscheibe (1; 10) mindestens zwei Ausnehmungen (14, 15; 140) aufweist und mindestens zwei zweite Strahlenschutzscheiben (2) vorgesehen sind, die relativ zu der ersten Strahlenschutzscheibe (1; 10) derart verstellbar sind, dass mit jeweils einer zweiten Strahlenschutzscheibe (2) eine der mindestens zwei Ausnehmungen (14, 15; 140) abdeckbar ist.

4. Strahlenschutzvorrichtung (3) nach einem der Ansprüche 1 bis 3 mit einer Drehbefestigung (5) zum relativen Verstellen der mindestens einen zweiten Strahlenschutzscheibe (2) gegenüber der ersten Strahlenschutzscheibe (1; 10) um eine Drehachse, wobei mehrere zweite Strahlenschutzscheiben (2) vorzugsweise um eine gemeinsame Drehachse verstellbar sind.

5. Strahlenschutzvorrichtung (3) nach einem der vorangehenden Ansprüche, wobei die erste Strahlenschutzscheibe (1; 10) eine rechteckförmige Fläche aufweist, wobei vorzugsweise die Ecken der rechteckförmigen Fläche abgerundet sind, und wobei besonders bevorzugt ein erstes Paar einander diagonal gegenüberliegende Ecken eine erste Abrundung (12; 120) aufweisen und ein zweites Paar einander diagonal gegenüberliegende Ecken eine zweite und dritte Abrundung (13a, 13b; 130a, 130b) aufweisen, wobei die erste Abrundung (12; 120) einen größeren Radius aufweist als die zweite und dritte Abrundung (13a, 13b; 130a, 130b).

6. Strahlenschutzvorrichtung (3) nach Anspruch 5, wobei die zweite Strahlenschutzscheibe (2) eine rechteckförmige Fläche aufweist, die kleiner als die rechteckförmige Fläche der ersten Strahlenschutzscheibe (1; 10) ist, wobei vorzugsweise eine Seite der Fläche mindestens teilweise schräg verläuft und wobei die Fläche und Form der zweiten Strahlenschutzscheibe (2) an die jeweils abzudeckende Ausnehmung (14, 15; 140) in der ersten Strahlenschutzscheibe (1; 10) angepasst ist, und vorzugsweise die zweite Strahlenschutzscheibe (2) über den Rand der abzudeckenden Ausnehmung (14, 15; 140) hinausragt.

7. Strahlenschutzvorrichtung (3) nach einem der vorangehenden Ansprüche, wobei die erste Strahlenschutzscheibe (1; 10) und die zweite Strahlenschutzscheibe (2) aus einem transparenten Material, vorzugsweise aus Bleiacrylglas bestehen.

## Claims

1. A radiation protection device (3) for shielding radiation, in particular X-ray radiation, emitted by a radiation source, comprising a first radiation protection panel (1; 10), wherein the first radiation protection panel (1; 10) comprises at least one first recess (14, 15; 140), and at least one second radiation protection panel (2), which is adjustable relative to the first radiation protection panel (1; 10) in such a way that at least one recess (14, 15; 140) can be covered by the second radiation protection panel (2), and comprising a connection element (5) for attachment to a holding element (4), wherein the holding element (4) can be attached to a stationary means in a room or to an examination device or to a support of an examination table, **characterized in that** the first radiation protection panel (1; 10) and the second radiation protection panel (2) are attached to the connection element (5) and the second radiation protection panel (2) is attached to the connection element (5) in a rotatable manner.

2. The radiation protection device (3) according to claim 1, wherein the second radiation protection panel (2) is configured and adjustable relative to the first radiation protection panel (1; 10) in such a way that by means of the second radiation protection panel (2) selectively either one or two or more of the recesses (14, 15; 140) present in the first radiation protection panel (1; 10) can be covered.

3. The radiation protection device (3) according to claim 1, wherein the first radiation protection panel (1; 10) comprises at least two recesses (14, 15; 140) and at least two second radiation protection panels (2) are provided, which are adjustable relative to the first radiation protection panel (1; 10) in such a way that by means of a respective second radiation protection panel (2) one of the at least two recesses (14, 15; 140) can be covered.

4. The radiation protection device (3) according to any one of claims 1 to 3, comprising a rotational fixation (5) for adjusting the at least one second radiation protection panel (2) relative to the first radiation protection panel (1; 10) about a rotational axis, wherein a plurality of second radiation protection panels (2) are preferably adjustable about a common rotational axis.

5. The radiation protection device (3) according to any one of the preceding claims, wherein the first radiation protection panel (1; 10) has a rectangular surface, wherein preferably the corners of the rectangular surface are rounded, and wherein particularly preferably a first pair of diagonally opposite corners has a first curvature (12; 120) and a second pair of diagonally opposite corners has a second and a third curvature (13a, 13b; 130a; 130b), wherein the first curvature (12; 120) has a larger radius than the second and third curvatures (13a; 13b; 130a, 130b).

6. The radiation protection device (3) according to claim 5, wherein the second radiation protection panel (2) has a rectangular surface which is smaller than the rectangular surface of the first radiation protection panel (1; 10), wherein preferably one side of the surface is at least partially inclined and wherein the surface and shape of the second radiation protection panel (2) are adapted to the respective recess (14, 15; 140) in the first radiation protection panel (1; 10) which should be covered, and preferably the second radiation protection panel (2) extends beyond the edge of the recess (14, 15; 140) to be covered.

7. The radiation protection device (3) according to any one of the preceding claims, wherein the first radiation protection panel (1; 10) and the second radiation protection panel (2) are made from a transparent material, preferably from lead acrylic glass.

## Revendications

1. Ensemble de protection contre les rayonnements ou radiations (3), destiné à former écran à l'encontre d'un rayonnement émis par une source de rayonnement, notamment un rayonnement X, comprenant une première vitre de protection contre les rayonnements (1; 10), la première vitre de protection contre les rayonnements (1; 10) présentant au moins un évidement (14, 15; 140), l'ensemble comprenant également au moins une deuxième vitre de protection contre les rayonnements (2), qui peut être déplacée par rapport à la première vitre de protection contre les rayonnements (1; 10) de façon à pouvoir recouvrir au moins un évidement (14, 15; 140) avec la deuxième vitre de protection contre les rayonnements (2), et l'ensemble comprenant en outre un élément de raccordement (5) pour le montage sur une pièce de support (4), la pièce de support (4) pouvant être fixée à une installation en position fixe, dans un local ou bien sur un appareil d'examen ou bien encore sur une structure d'une table d'examen,
**caractérisé en ce que** la première vitre de protection contre les rayonnements (1; 10) et la deuxième vitre de protection contre les rayonnements (2) sont fixées à l'élément de raccordement (5), et la deuxième vitre de protection contre les rayonnements (2) est fixée de manière rotative à l'élément de raccordement (5).

2. Ensemble de protection contre les rayonnements (3) selon la revendication 1, dans lequel la deuxième vitre de protection contre les rayonnements (2) est configurée et déplaçable par rapport à la première vitre de protection contre les rayonnements (1; 10) de manière telle, qu'il soit possible de recouvrir avec la deuxième vitre de protection contre les rayonnements (2), au choix, un ou deux ou plusieurs évidements (14, 15; 140) existant sur la première vitre de protection contre les rayonnements (1; 10).

3. Ensemble de protection contre les rayonnements (3) selon la revendication 1, dans lequel la première vitre de protection contre les rayonnements (1; 10) présente au moins deux évidements (14, 15; 140), et dans lequel sont prévues au moins deux deuxièmes vitres de protection contre les rayonnements (2), qui peuvent être déplacées par rapport à la première vitre de protection contre les rayonnements (1; 10) de manière à pouvoir recouvrir, à l'aide de respectivement une deuxième vitre de protection contre les rayonnements (2), l'un desdits au moins deux évidements (14, 15; 140).

4. Ensemble de protection contre les rayonnements (3) selon l'une des revendications 1 à 3, comprenant une fixation de rotation (5) pour le déplacement relatif de ladite au moins une deuxième vitre de protection contre les rayonnements (2) par rapport à la première vitre de protection contre les rayonnements (1; 10) autour d'un axe de rotation, plusieurs deuxièmes vitres de protection contre les rayonnements (2) étant de préférence déplaçables autour d'un axe de rotation commun.

5. Ensemble de protection contre les rayonnements (3) selon l'une des revendications précédentes, dans lequel la première vitre de protection contre les rayonnements (1; 10) présente une surface de forme rectangulaire, dans lequel les sommets de la surface rectangulaire sont de préférence arrondis, et dans lequel de manière particulièrement préférée, une première paire de sommets diagonalement opposés présentent un premier arrondi (12; 120), et une deuxième paire de sommets diagonalement opposés présentent un deuxième et un troisième arrondi (13a, 13b; 130a, 130b), le premier arrondi (12; 120) présentant un rayon plus grand que le deuxième et le troisième arrondi (13a, 13b; 130a, 130b).

6. Ensemble de protection contre les rayonnements (3) selon la revendication 5, dans lequel la deuxième vitre de protection contre les rayonnements (2) présente une surface de forme rectangulaire, qui est plus petite que la surface rectangulaire de la première vitre de protection contre les rayonnements (1; 10), dans lequel de préférence un côté de ladite surface s'étend au moins partiellement de manière oblique, et dans lequel la surface et la forme de la deuxième vitre de protection contre les rayonnements (2) sont adaptées à l'évidement (14, 15; 140) respectif de la première vitre de protection contre les rayonnements (1; 10) à couvrir, et la deuxième vitre de protection contre les rayonnements (2) dépasse de préférence du bord de l'évidement (14, 15; 140) à couvrir.

7. Ensemble de protection contre les rayonnements (3) selon l'une des revendications précédentes, dans lequel la première vitre de protection contre les rayonnements (1; 10) et la deuxième vitre de protection contre les rayonnements (2) sont réalisées en un matériau transparent, de préférence un verre acrylique au plomb.
